# EUROPEAN PATENT APPLICATION

(11) **EP 4 273 120 A1**
(43) Date of publication of application: **08.11.2023**
(21) Application number: 21923447.3
(22) Date of filing: 23.12.2021
(51) Int. Cl.: C07C 235/84, C07D 207/08, A61K 31/166, A61K 31/40, A61P 29/00

(54) **NOVEL COMPOUND EXHIBITING ANTI-INFLAMMATORY ACTIVITY AS P38 MAP KINASE INHIBITOR**

(30) Priority: 29.01.2021 KR 20210013198
(71) Applicant: Prazertherapeutics Inc., Seoul 02455 (KR)
(72) Inventor: INN, Kyung-Soo, Goyang-si, Gyeonggi-do 10491 (KR); KIM, Nam-Jung, Seoul 06356 (KR); LEE, Jong Kil, Seoul 02567 (KR); KIM, Ga Yeong, Seoul 05837 (KR); KIM, Kyeojin, Seoul 06356 (KR); KIM, Donghwan, Seoul 02811 (KR); DO, Jimin, Seoul 05354 (KR); SONG, Chaewon, Seoul 05245 (KR); LEE, Na-Rae, Namyangju-si, Gyeonggi-do 12113 (KR)
(74) Representative: Roos, Peter
(86) International application number: PCT/KR2021/019686
(87) International publication number: WO 2022/164041

(57) **Abstract**

The present invention relates to a novel compound exhibiting anti-inflammatory activity, and the compound of the present invention plays a key role in the generation of pro-inflammatory cytokines, thereby having excellent inhibitory activity against p38 MAPK, which is known to cause inflammatory diseases, and thus can be effectively used as an agent for treating inflammatory diseases.

## Description

### Technical Field

The present disclosure relates to a novel compound exhibiting anti-inflammatory activity, wherein the compound of the present disclosure particularly has an excellent p38 MAP kinase inhibitory effect and thus may be useful as an agent for treating inflammatory diseases.

### Background Art

p38 is a type of serine/threonine kinase that belongs to the mitogen-activated protein kinase (MAPK) family. The MAPK pathway is activated by various external stimuli of cells, and conversion is involved to cause various intracellular reactions via phosphorylation of the specific matrix of the cell, wherein subtypes of MAPK include JNK and ERK in addition to p38. MAPK is a protein kinase that is activated by activating stimuli of insulin, mitogens, growth factors, and immune cells and takes an important position in the signaling pathway to transmit signals from receptors existing on the cell surface into the cell.

The p38 signaling system regulates processes such as metabolism and cell death and undergoes a series of phosphorylation cascade where upper-level kinases transfer phosphates to lower-level kinases. Various forms of p38 MAPK have been identified, including p38-α, p38-β, p38-γ, and p38-δ, which are activated such as osmotic shock, inflammatory cytokines, lipopolysaccharide (LPS), ultraviolet (UV), and growth factors (GFs).

Inflammation is known to be caused by a variety of causes, and according to several studies, it has been found that MAPK may be activated by various stress stimuli, and especially p38 MAPK plays a crucial role in generation of pro-inflammatory cytokines such as TNF-α, IL-1β, and IL-6 and is known to cause inflammatory diseases (NATURE REVIEWS, Drug discovery (2003), Vol.2, 717-726). Accordingly, several small molecules have been developed as p38 MAPK inhibitors (Korean Patent No. 10-1879481), and various small molecule inhibitors of p38 inhibit the synthesis of IL-1 and TNF in human monocytes at a low concentration to function as an inflammatory modulator, and it was revealed that inhibition of these cytokines may regulate, alleviate, or mitigate inflammatory responses.

In this regard, the inventors of the present disclosure have completed the present disclosure through research on novel compounds having anti-inflammatory activity through inhibition of p38 MAPK.

### Disclosure of the Invention

### Technical Goals

An object of the present disclosure is to provide a novel compound having excellent anti-inflammatory efficacy by effectively inhibiting p38 MAP kinase, for effective treatment of inflammatory diseases.

### Technical Solutions

In order to achieve the above object, the present disclosure provides a compound represented by the following Chemical Formula 1 or a pharmaceutically acceptable salt thereof.

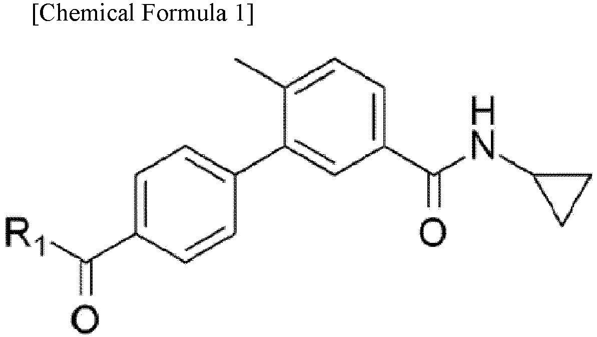

In the above Chemical Formula,
R₁ is alkyl, cycloalkyl, cycloalkenyl, or heterocycloalkyl,
the R₁ may be substituted with one or more substituents selected from the group consisting of -OH, alkoxy, -alkylalkoxy, -O-(CH₂)ₘ-OH, and -O-(CH₂)ₘ-O- alkyl, and
m is an integer from 1 to 6.

In one embodiment, R₁ may be C₁-C₆alkyl, C₃-C₁₀cycloalkyl, C₃-C₁₀cycloalkenyl, or C₃-C₁₀heterocycloalkyl, and the R₁ may be -OH, C₁-C₆alkoxy, -C₁-C₆alkylC₁-C₆alkoxy, -O-(CH₂)m-OH, and -O-(CH₂)m-O-C₁-C₆alkyl.

In one embodiment, R₁ may be -C₁-C₆alkyl-OH; -C₁-C₆alkyl-O-C₁-C₆alkyl; C₃-C₇cycloalkyl substituted or unsubstituted with -OH, -O-(CH₂)m-OH, or -O-(CH₂)m-O-C₁-C₆alkyl; C₃-C₇cycloalkenyl; or C₃-C₇heterocycloalkyl substituted or unsubstituted with -C₁-C₆alkylC₁-C₆alkoxy.

In one embodiment, R₁ is -C₁-C₆alkyl-OH; -C₁-C₆alkyl-O-C₁-C₆alkyl; C₃-C₇cycloalkyl substituted or unsubstituted with -OH, -O-(CH₂)ₘ-OH, or -O-(CH₂)ₘ-O-C₁-C₆alkyl; C₃-C₇cycloalkenyl; or C₃-C₇heterocycloalkyl including one nitrogen atom substituted or unsubstituted with -C₁-C₆alkylC₁-C₆alkoxy.

In one embodiment, the compound of the present disclosure may be a compound selected from the group consisting of the following compounds or a pharmaceutically acceptable salt thereof.

| No. | Chemical name | Structural formula |
|---|---|---|
| 1 | N-cyclopropyl-4'-(2-hydroxy-2-methylpropanoyl)-6-methyl-[1,1'-biphenyl]-3-carboxamide | 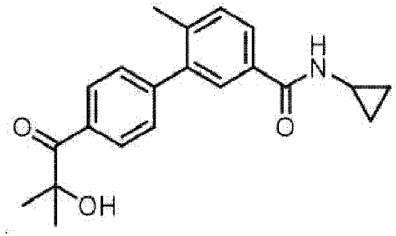 |
| 2 | N-cyclopropyl-4'-(4-methoxy-2-methylbutanoyl)-6-methyl-[1,1'-biphenyl]-3-carboxamide | 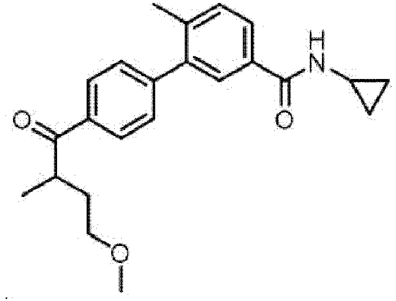 |
| 3 | N-cyclopropyl-4'-(5-methoxy-2-methylpentanoyl)-6-methyl-[1,1'-biphenyl]-3-carboxamide | 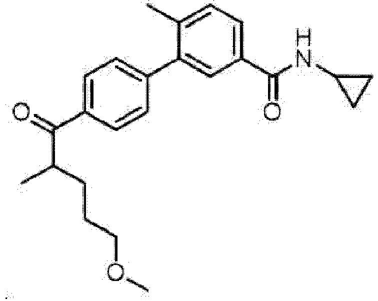 |
| 4 | 4'-(cyclopropanecarbonyl)-N-cyclopropyl-6-methyl-[1,1'-biphenyl]- 3-carboxamide | 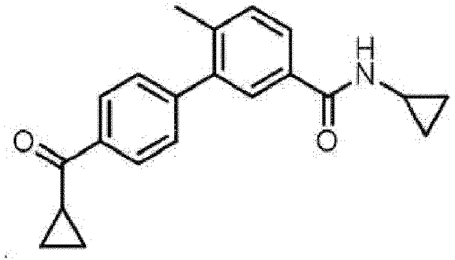 |
| 5 | 4'-(cyclobutanecarbonyl)-N-cyclopropyl-6-methyl-[1,1'-biphenyl]- 3-carboxamide | 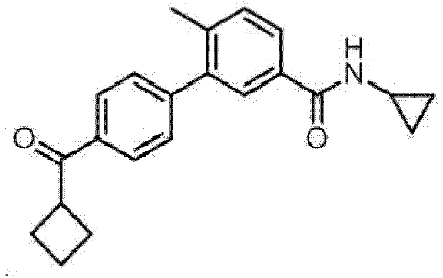 |
| 6 | 4'-(cyclopentanecarbonyl)-N-cyclopropyl-6-methyl-[1,1'-biphenyl]- 3-carboxamide | 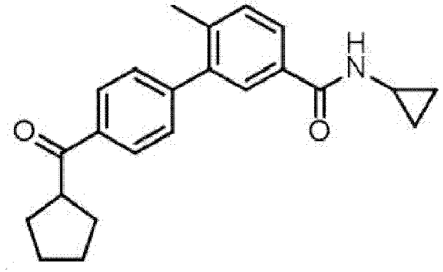 |
| 7 | 4'-(cyclohexanecarbonyl)-N-cyclopropyl-6-methyl-[1,1'-biphenyl]- 3-carboxamide | 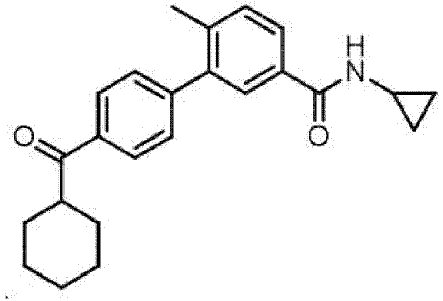 |
| 8 | 4'-(cycloheptanecarbonyl)-N-cyclopropyl-6-methyl-[1,1'-biphenyl]- 3-carboxamide | 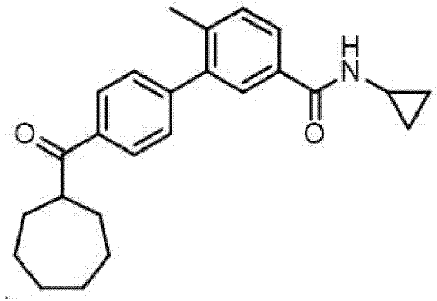 |
| 9 | 4'-(cyclopent-3-enecarbonyl)-N-cyclopropyl-6-methyl-[1,1'-biphenyl]- 3-carboxamide | 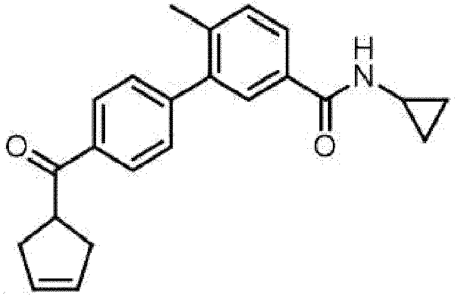 |
| 10 | N-cyclopropyl-4'-(3-hydroxycyclopentanecarbonyl)-6-methyl-[1,1'-biphenyl]-3-carboxamide | 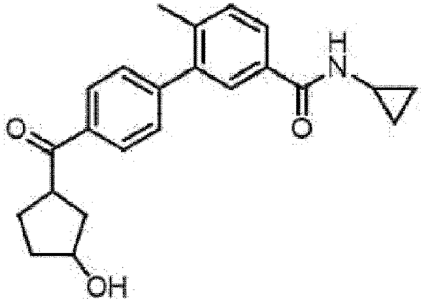 |
| 11 | N-cyclopropyl-4'-(3 -(2-hydroxyethoxy)cyclopentanecarbonyl) -6-methyl-[1,1'-biphenyl]-3- carboxamide | 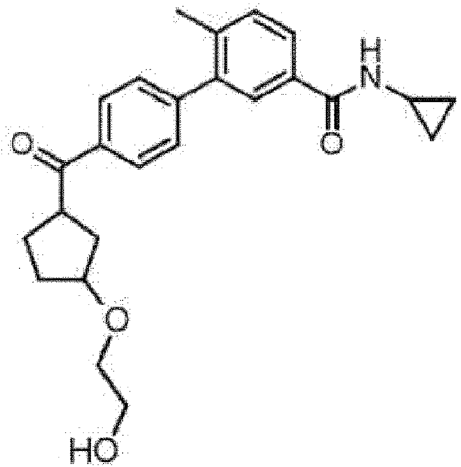 |
| 12 | N-cyclopropyl-4'-(3 -(2-methoxyethoxy)cyclopentane-1- carbonyl)-6-methyl-[1,1'-biphenyl]-3-carboxamide | 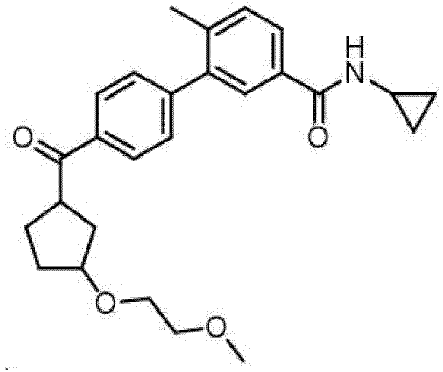 |
| 13 | N-cyclopropyl-4'-(2-hydroxypropanoyl)-6-methyl-[1,1'-biphenyl]-3-carboxamide | 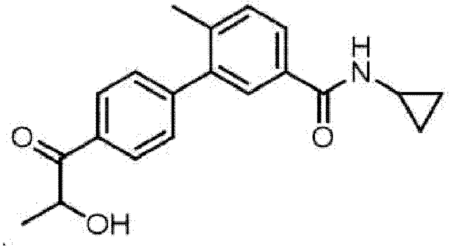 |
| 14 | N-cyclopropyl-6-methyl-4'-(pyrrolidine-3-carbonyl)-[1,1'-biphenyl]-3-carboxamide | 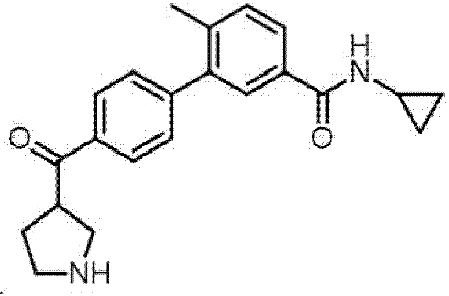 |
| 15 | N-cyclopropyl-4'-(1-(2-methoxyethyl)pyrrolidine-3-carbonyl)-6-methyl-[1,1'-biphenyl]-3-carboxamide | 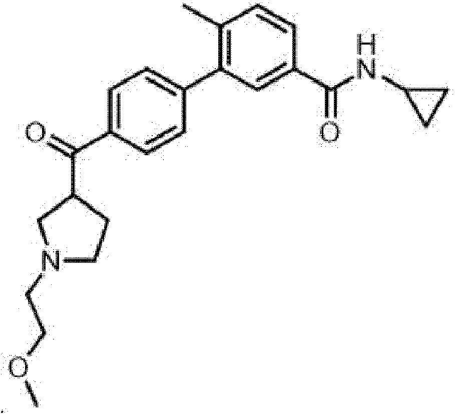 |

### Advantageous Effects

The compound of the present disclosure has excellent inhibitory efficacy against p38 MAPK, which is known to cause inflammatory diseases by playing a crucial role in generation of pro-inflammatory cytokines and thus may be useful as an agent for treating inflammatory diseases. Best Mode for Carrying Out the Invention

Hereinafter, the present disclosure will be described in more detail.

### Definition

The term "pharmaceutically acceptable salt" as used herein refers to a salt that is pharmaceutically acceptable and maintains preferred pharmacological activity of a parent compound. The salt is not particularly limited if they are pharmaceutically acceptable, for example, hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methanesulfonic acid, benzenesulfonic acid, toluenesulfonic acid, and naphthalenesulfonic acid.

The term "alkyl" as used herein refers to a hydrocarbon having primary, secondary, tertiary, or quaternary carbon atoms and includes a saturated aliphatic group that may be linear, branched, or cyclic, or a combination thereof. For example, an alkyl group may have 1 to 20 carbon atoms (i.e., C₁-C₂₀ alkyl), 1 to 10 carbon atoms (i.e., C₁-C₁₀ alkyl), or 1 to 6 carbon atoms (i.e., C₁-C₆ alkyl). Examples of suitable alkyl groups may include methyl (Me, -CH₃), ethyl (Et, - CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH2CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃), but are not limited thereto.

Moreover, the term "alkyl" used throughout the specification, example embodiments and claims is intended to include both unsubstituted and substituted alkyl groups.

The term "alkoxy" as used herein refers to a group having the formula -O-alkyl in that the alkyl group as defined above is bonded to a parent compound by an oxygen atom. Alkyl residues in an alkoxy group may have, for example, 1 to 20 carbon atoms (i.e., C₁-C₂₀ alkoxy), 1 to 12 carbon atoms (i.e., C₁-C₁₂ alkoxy), 1 to 10 carbon atoms (i.e., C₁-C₁₀ alkoxy), or 1 to 6 carbon atoms (i.e., C₁-C₆ alkoxy). Examples of suitable alkoxy groups may include methoxy (-O-CH₃ or -OMe), ethoxy (-OCH₂CH₃ or -OEt), and t-butoxy (-OC(CH₃)₃ or -O-tBu), but are not limited thereto.

The term "alkoxyalkyl" as used herein refers to an alkyl group substituted with an alkoxyl group and may be represented by the general formula -alkyl-O-alkyl.

"Alkenyl" is a hydrocarbon having primary, secondary, tertiary and/or quaternary carbon atoms, comprising linear, branched, and cyclic groups or a combination thereof, and possessing one or more unsaturated regions, i.e., carbon-carbon sp² double bonds. For example, the alkenyl group may have 2 to 20 carbon atoms (i.e., C₂-C₂₀ alkenyl), 2 to 12 carbon atoms (i.e., C₂-C₁₂ alkenyl), 2 to 10 carbon atoms (i.e., C₂-C₁₀ alkenyl), or 2 to 6 carbon atoms (i.e., C₂-C₆ alkenyl). Examples of suitable alkenyl groups may include vinyl (-CH=CH₂), allyl (-CH₂CH=CH₂), and 5-hexenyl (-CH₂CH₂CH₂CH₂CH=CH₂), but are not limited thereto.

"Alkynyl" is a hydrocarbon having primary, secondary, tertiary and/or quaternary carbon atoms, comprising linear, branched, and cyclic groups or a combination thereof, and possessing one or more carbon-carbon sp triple bonds. For example, the alkynyl group may have 2 to 20 carbon atoms (i.e., C₂-C₂₀ alkynyl), 2 to 12 carbon atoms (i.e., C₂-C₁₂ alkynyl), 2 to 10 carbon atoms (i.e., C₂-C₁₀ alkynyl), or 2 to 6 carbon atoms (i.e., C₂-C₆ alkynyl). Examples of suitable alkynyl groups may include acetylenic (-C=CH) and propargyl (-CH₂C≡CH), but are not limited thereto.

"Aryl" includes a substituted or unsubstituted, monovalent or divalent, aromatic hydrocarbon group in which each of the atoms in the ring is carbon and which is monocyclic, bicyclic, or polycyclic. Preferably, the aryl ring is a 6- to 20-cyclic ring, a 6- to 14-cyclic ring, a 6- to 10-cyclic ring, or more preferably a 6-cyclic ring. The aryl group may be a polycyclic ring system having two or more cyclic rings in which two or more carbons are common to two adjacent rings, wherein one or more of the rings may be aromatic, for example, the other cyclic rings may be cycloalkyl, cycloalkenyl, cycloalkynyl, aryl, heteroaryl, and/or heterocycloalkyl. Examples of aryl groups may include benzene, naphthalene, phenanthrene, anthracene, indene, indane, phenol, and aniline.

"Heteroaryl" refers to a substituted or unsubstituted, monovalent or divalent, aromatic group that contains one or more heteroatoms in the ring and is monocyclic, bicyclic or polycyclic. Non-limiting examples of heteroatoms that are suitable to be contained in aromatic rings include oxygen, sulfur, and nitrogen. In the polycyclic heteroaryl ring system, the cyclic system has two or more cyclic rings in which two or more carbons are common to two adjacent rings, wherein one or more of the rings may be heteroaromatic, for example, other cyclic rings may be cycloalkyl, cycloalkenyl, cycloalkynyl, aryl, heteroaryl, and/or heterocyclyl. Heteroaryl groups include, for example, benzofuran, benzothiophene, pyrrole, furan, thiophene, imidazole, indole, isoindole, isoxazole, isothiazole, oxazole, thiazole, quinoline, isoquinoline, pyrazole, pyridine, pyrazine, pyridazine, and pyrimidine (each of these may be substituted or unsubstituted).

"Cycloalkyl" refers to a non-aromatic, saturated or unsaturated, monovalent or divalent ring which may be monocyclic, bicyclic, or polycyclic while each of the atoms in the ring is carbon. The cycloalkyl group may have 3 to 10 carbon atoms as a monocycle, 7 to 12 carbon atoms as a bicycle, and about less than or equal to 20 carbon atoms as a polycycle. Monocyclic cycloalkyl has 3 to 10 cyclic atoms, more typically 5 or 6 cyclic atoms. Bicyclic cycloalkyl may have 7 to 12 cyclic atoms and may be a fused ring system, a spirocyclic ring system, or a bridged ring system. In an exemplary cycloalkyl group, atoms may be arranged in a bicyclo[4,5], [5,5], [5,6], or [6,6] system. In certain example embodiments, the cycloalkyl contains 3 to 20 atoms, or 3 to 10 atoms, or more preferably 3 to 7 atoms. Examples of cycloalkyl may include cyclopropyl, cyclobutyl, cyclopentyl, cyclohexyl, cycloheptyl, and cyclooctyl. Unless otherwise specified, cycloalkyl may be substituted by one or more substituents described herein.

"Cycloalkenyl" refers to a non-aromatic monocyclic or polycyclic hydrocarbon containing at least one carbon-carbon double bond. In some example embodiments, the cycloalkenyl includes 3 to 20 carbon atoms, 3 to 10 carbon atoms, and 3 to 7 carbon atoms. Cycloalkenyl includes monocyclic and polycyclic groups (including fused, bridged, and spirocycle). Examples of suitable cycloalkenyl groups may include cyclopentenyl (-C₅H₇), but are not limited thereto.

"Heterocycloalkyl" refers to a saturated or unsubstituted, monovalent or divalent, or saturated or partially saturated, non-aromatic ring structure in which a ring structure includes one or more heteroatoms, preferably 1 to 4 heteroatoms, more preferably 1 to 2 heteroatoms, preferably a 3- to 10-cyclic ring, more preferably a 3- to 7-cyclic ring.

The terms "halo" and "halogen" as used herein refer to halogens and include chloro, fluoro, bromo, and iodo.

"Haloalkyl" is an alkyl group in which one or more of hydrogen atoms in the alkyl group as defined above have been replaced by a halogen atom. Alkyl residues in the haloalkyl group may have 1 to 20 carbon atoms (i.e., C₁-C₂₀ haloalkyl), 1 to 12 carbon atoms (i.e., C₁-C₁₂ haloalkyl), 1 to 10 carbon atoms (i.e., C₁-C₁₀ haloalkyl), or 1 to 6 carbon atoms (i.e., C₁-C₆ haloalkyl). Exemplary haloalkyl groups may include -CF₃, -CHF₂, -CFH₂, and -CH₂CF₃, but are not limited thereto.

The term "Boc" refers to a tert-butoxycarbonyl group.

In certain example embodiments, the compound of the disclosure may be racemic. In certain example embodiments, one enantiomer of the compound of the present disclosure may be enhanced. For example, the compound of the present disclosure may have at least 30% ee, 40% ee, 50% ee, 60% ee, 70% ee, 80% ee, 90% ee, or even 95% ee or more.

A single bond shown herein without stereochemistry does not represent the stereochemistry of the compound, but a compound of the present disclosure may have one or more stereocenters within a structure thereof. These stereocenters may exist in R or S configuration, and the R and S expressions are used according to rules described in the document [Pure Appl. Chem. (1976), 45, 11-30]. The present disclosure considers all stereoisomeric forms of compounds, salts thereof, prodrugs, or mixtures, such as enantiomer and diastereoisomer forms (including all possible mixtures of stereoisomers).

Certain compounds of the present disclosure have more than one stereocenter. Thus, one or more diastereoisomers of the compound of the present disclosure may be enhanced. For example, the compound of the present disclosure may have at least 30% de, 40% de, 50% de, 60% de, 70% de, 80% de, 90% de, or even 95% de or more.

The term "Cx-y" or "Cx-Cy" is considered to include groups containing x to y carbons in the chain, when used along with chemical residues such as alkyl, alkenyl, or alkynyl. For C₀ alkyl, if the group is present at a terminal position, it represents hydrogen, and if it is inside, it represents bonding. For example, C₁-C₂₀ alkyl groups contain 1 to 20 carbon atoms in the chain.

Compounds according to the present disclosure may be prepared using modifications for certain synthetic protocols described below that are well known to those skilled in the art from readily available starting materials.

### Modes for Carrying Out the Invention

### [Manufacturing Example]

Instruments and reagents used in a synthesis method of compounds according to the present disclosure

All starting materials and reagents used in the synthesis of compounds of the present disclosure were purchased from Aldrich, Alfa Aesar, and TCI, and used without a separate purification process. Various glass used in the reaction were used in a state of being dried in an oven at 80°C, and the reaction was carried out by injecting argon gas or nitrogen gas to block air and moisture.

For flash column chromatography of the present disclosure, silica gel 60 (230-400 mesh) was used by appropriately controlling solvents such as hexane, ethyl acetate, dichloromethane, and methanol. 0.25 mm silica gel plates were used for analytical thinlayerchromatography.

The ¹H-NMR spectra and ¹³C-NMR spectra of the present disclosure were analyzed using a Bruker Avance 400 (400 MHz for 1H; 100 MHz for 13C) spectrometer or a VARIAN VNMRS 500 (500 MHz for 13C) spectrometer. ¹H and ¹³C NMR chemical shift values were expressed in ppm using tetramethylsilane (TMS) as an internal reference. NMR signals were expressed as m (multiplet), s (singlet), d (doublet), t (triplet), q (quartet), quin (quintet), bs (broad singlet), bd (broad doublet), dd (doublet of doublets), dt (doublet of triplets), and dq (doublet of quartets), and the coupling constant was expressed in hertz (Hz).

The low resolution mass spectra and high resolution mass spectra of the present disclosure were measured using the VG Trio-2 GC-MS or JEOL JMS-700 instrument.

In one example embodiment, intermediate compounds 16 to 17 of the present disclosure may be prepared according to the following scheme 1.

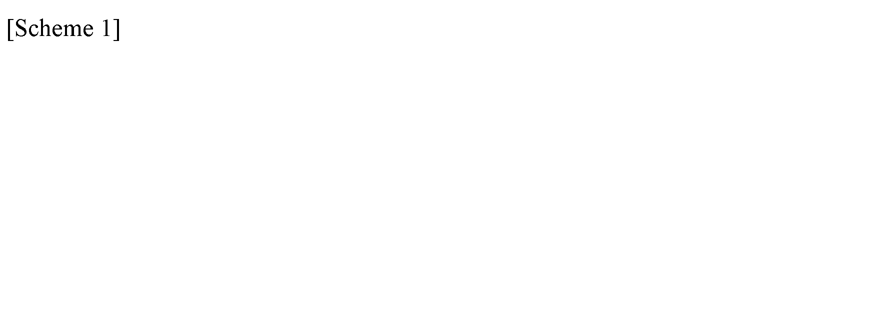

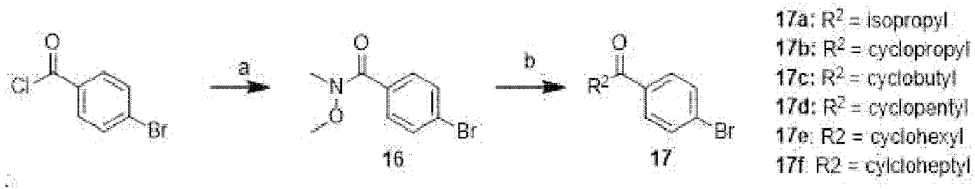

Reagents and conditions: (a) N,O-dimethylhydroxylamine, hydrochloric acid, triethylamine, dichloromethane, r.t. (room temperature), 100%; (b) RMgBr or RMgCl, tetrahydrofuran (dry), r.t., 30-100%.

Commercially available 4-bromobenzoyl chloride was used to carry out synthesis according to Scheme 1 above. According to Scheme 1-a, weinreb amide was formed to obtain compound 16, and compounds 17a to 17f were obtained using suitable alkyl Grignard reagents.

In one example embodiment, intermediate compound 18 of the present disclosure may be prepared according to the following scheme 2.

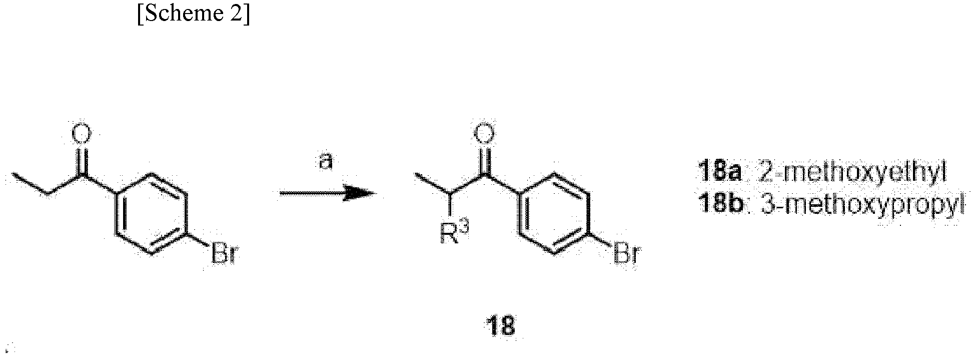

Reagents and conditions: (a) 4'-bromopropiophenone, RBr, t-BuOK, t-BuOH, reflux, 9-30%.

Using commercially available 4'-bromopropiophenone, an appropriate bromo alkyl reagent was added to proceed an alkylation reaction at the carboxyl α position, thereby obtaining compounds 18a and 18b.

In one example embodiment, intermediate compounds 19 to 23 of the present disclosure may be prepared according to the following scheme 3.

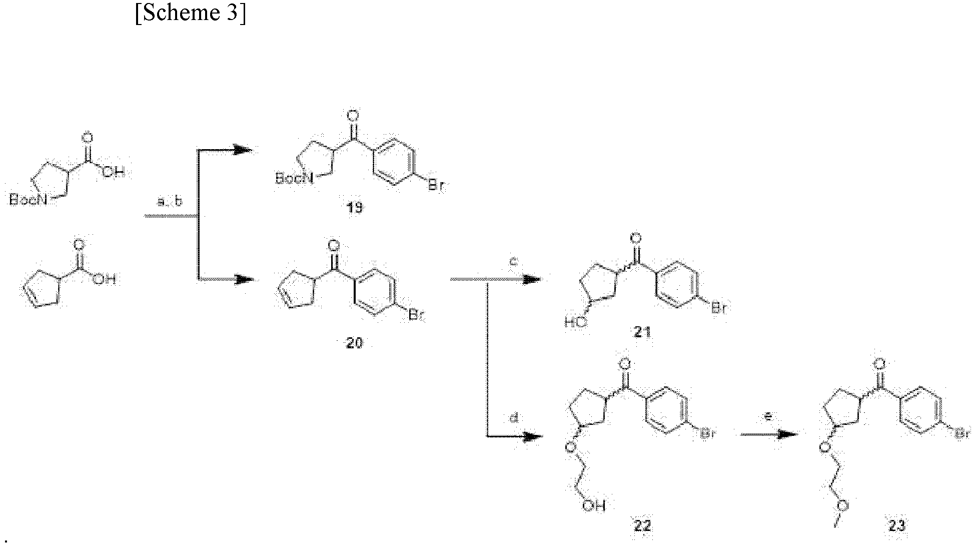

Reagents and conditions: (a) N,O-dimethylhydroxylamine, hydrochloric acid, EDCI (1-ethyl-3-(3-dimethylaminopropyl)carbodiimide), 4-dimethylaminopyridine, dimethylformamide, r.t., 78-99%; (b) 1,4-dibromobenzene, n-butyllithium, tetrahydrofuran (dry), -78°C, 52-83%; (c) 1M borane (in THF), tetrahydrofuran (dry), r.t., 2.5h; water, sodium hydroxide, hydrogen peroxide, r.t., 48%; (d) Hg(OCOCF₃)₂, ethylene glycol, r.t., 30min; sodium hydroxide, sodium borohydride, r.t., 70%; (e) t-BuOK, MeI, THF; 32%.

Using commercially available 1-(tert-butoxycarbonyl)pyrrolidine-3-carboxylic acid or cyclopentene carboxylic acid as a starting material, Weinreb amide was formed through EDC coupling, and then dibromobenzene was subjected to a substitution reaction under n-butyllithium condition to obtain compounds 19 and 20. In addition hydroxycyclopentyl derivative compound 21 through a hydroboration reaction using compound 20 or hydroxyethoxycyclopentyl derivative compound 22 through an alkoxymercuration reaction was synthesized and obtained as diastereomeric mixture state, respectively. In addition, after dissolving compound 22 in THF, t-BuOK was added dropwise and MeI was added to obtain compound 23.

In one example embodiment, intermediate compound 24 of the present disclosure may be prepared according to the following scheme 4.

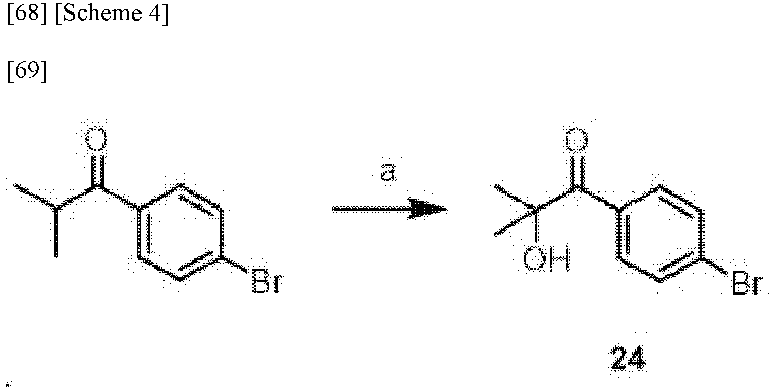

Reagents and conditions: (a) 1-(4-bromophenyl)-2-methylpropan-1-one, N-bromosuccinimide, DMSO, reflux, 70%.

Hydroxylation was carried out at a carboxyl α position using commercially available 1-(4-bromophenyl)-2-methylpropan-1-one to obtain intermediate compound 24.

In one example embodiment, compounds 1 to 12 of the present disclosure may be prepared according to the following scheme 5.

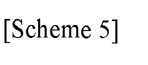

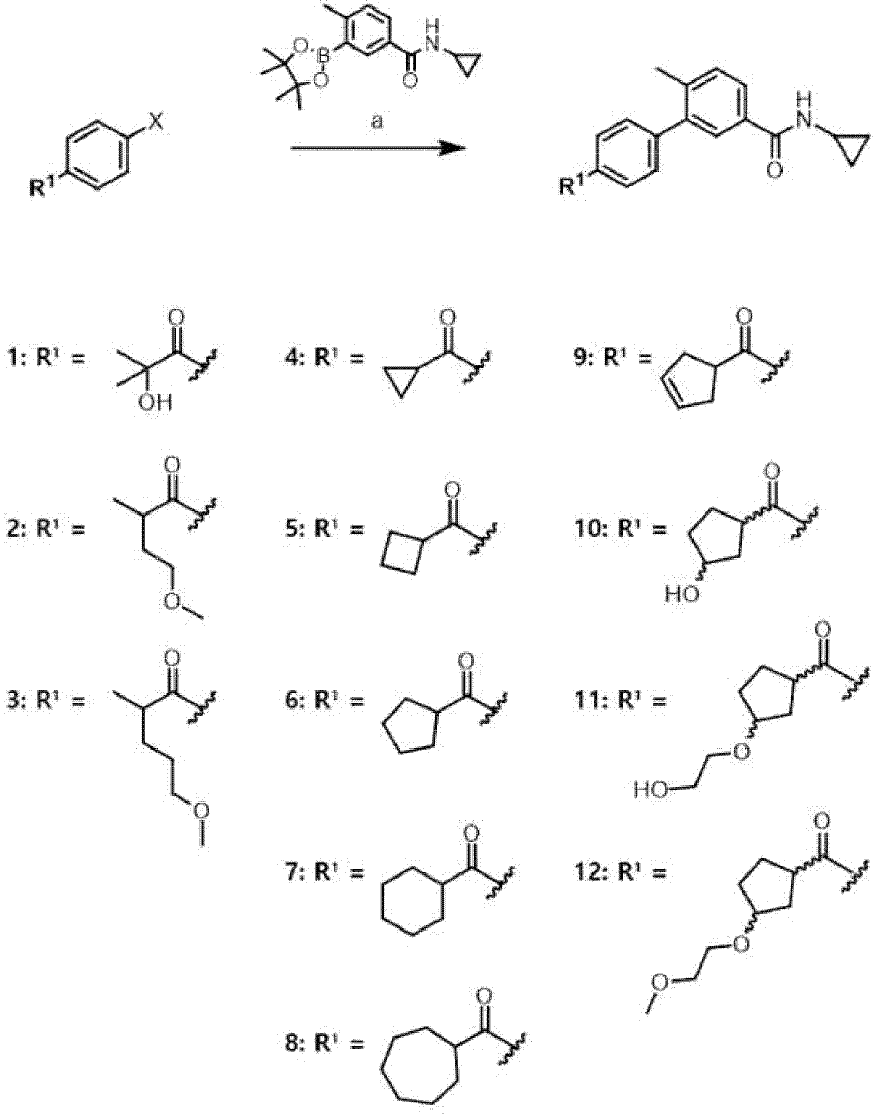

Reagents and conditions: (a) n-cyclopropyl-4-methyl-3-(4,4,5,5-tetramethyl-1,3,2-dioxaborolan-2-yl)-benzamide; tetrakis(triphenylphosphine)palladium; potassium carbonate; dimethylformamide 120°C or isopropyl alcohol 90°C; 10-72%.

In one example embodiment, compound 13 of the present disclosure may be prepared according to the following scheme 6.

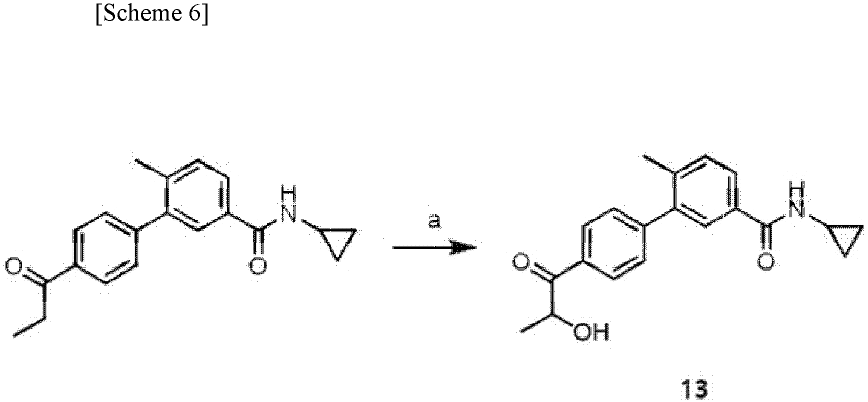

Reagents and conditions: (a) lithium hydroxide, tetrahydrofuran/methanol/water, r.t., 88%

In one example embodiment, compounds 14 and 15 of the present disclosure may be prepared according to the following scheme 7.

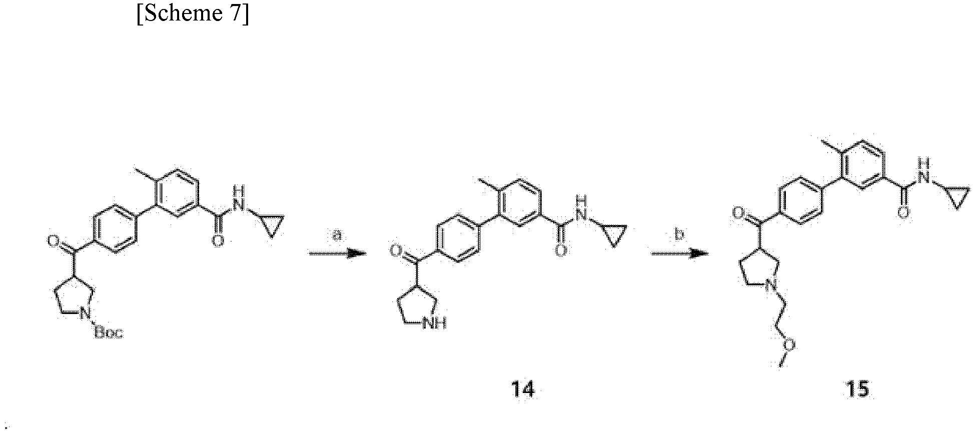

Reagents and conditions: (a) trifluoroacetic acid, dichloromethane, r.t., 41%; (b) 2-bromoethylmethylether, potassium carbonate, dimethylformamide, 50°C, 19%

The present disclosure will be described in detail with reference to the following Examples, but the scope of the present disclosure is not limited thereto.

### [Example 1]

### Synthesis of N-cyclopropyl-4'-(2-hydroxy-2-methylpropanoyl)-6-methyl-[1,1'-biphenyl]-3-carboxamide (compound 1)

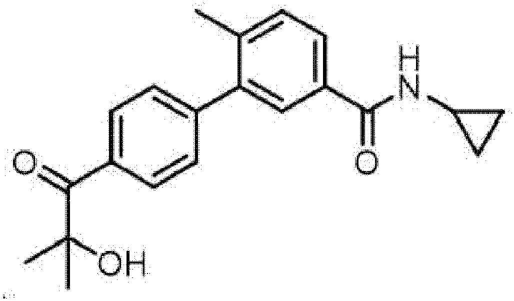

In the presence of intermediate compound, 1-propaneone and 1-(4-bromophenyl)-2-hydroxy-2-methyl (compound 22) (27.9 mg, 0.103 mmol), N-cyclopropyl-4-methyl-3-(4,4,5,5-tetramethyl-1,3,2-dioxaborolan-2-yl)benzamide (47 mg, 0.180 mmol), tetrakis(triphenylphosphine)palladium (14 mg, 0.012 mmol), and potassium carbonate (34 mg, 0.240 mmol) were dissolved in isopropyl alcohol (3 mL), and reflux reaction was carried out under argon gas condition. When it was observed that the starting material disappeared by thin-layer chromatography, the temperature was lowered to room temperature, the reaction was stopped using 2N-hydrochloric acid, filtration was performed using celite, and then the filtrate was extracted with ethyl acetate and water. Magnesium sulfate was added to the obtained organic layer to perform dehydration, and then the solvent of the filtrate obtained by filtering under reduced pressure was removed and concentrated. Compound 1 was then finally obtained by column chromatography (22mg, 66%); ¹H-NMR (400 MHz, CDCl₃) δ 8.09 (d, J = 8 Hz, 2H), 7.66 (dd, J = 8 Hz, 1H), 7.60-7.59 (m, 1H), 7.41 (d, J = 8 Hz, 2H), 7.33 (d, J = 8 Hz, 1H), 6.29 (s, 1H), 4.09 (s, 1H), 2.92-2.86 (m, 1H), 2.30 (s, 3H), 1.68 (s, 6H), 0.89-0.83 (m, 2H), 0.63-0.58(m, 2H).

### [Example 2]

### Synthesis of N-cyclopropyl-4'-(4-methoxy-2-methylbutanoyl)-6-methyl-[1,1'-biphenyl]-3-carboxamide (compound 2)

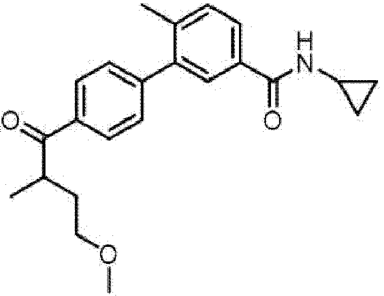

Intermediate compound 1-(4-bromophenyl)-4-methoxy-2-methylbutan-1-one (compound 18a) (21.9 mg, 0.081 mmol) was used as the starting material to finally obtain compound 2 through the same method as for compound 1 (10.0 mg, 34%); ¹H-NMR (400 MHz, CDCl₃) δ 8.03 (d, J = 8 Hz, 2H), 7.66 (dd, J = 8 Hz, 1H), 7.60-7.58 (m, 1H), 7.40 (d, J = 8 Hz, 2H), 7.32 (d, J = 8 Hz, 1H), 6.32 (bs, 1H), 3.77-3.68 (m, 1H), 3.50-3.43 (m, 1H), 3.41-3.34 (m, 1H), 3.29 (s, 3H), 2.93-2.85 (m, 1H), 2.29 (s, 3H), 1.75-1.66 (m, 2H), 1.24 (d, J = 7.2 Hz, 3H), 0.88-0.83 (m, 2H), 0.63-0.58 (m, 2H).

### [Example 3]

### Synthesis of N-cyclopropyl-4'-(5-methoxy-2-methylpentanoyl)-6-methyl-[1,1'-biphenyl]-3-carboxamide (compound 3)

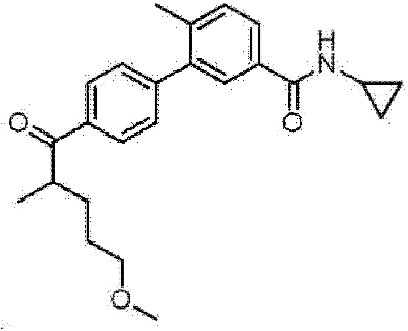

Intermediate compound 1-(4-bromophenyl)-5-methoxy-2-methylpentan-1-one (compound 18b) (20.7 mg, 0.073 mmol) was used as the starting material to finally obtain compound 3 through the same method as for compound 1 (10.0mg, 36%); ¹H-NMR (400 MHz, CDCl₃) δ 8.02 (d, J = 8 Hz, 2H), 7.66 (dd, J = 8 Hz, 1H), 7.60-7.57 (m, 1H), 7.41 (d, J = 8 Hz, 2H), 7.33 (d, J = 7.6 Hz, 1H), 6.27 (bs, 1H), 3.58-3.49 (m, 1H), 3.40-3.37 (m, 2H), 3.31 (s, 3H), 2.93-2.86 (m, 1H), 2.29 (s, 3H), 1.96-1.87 (m, 2H), 1.67-1.53 (m, 2H), 1.25 (s, 3H), 0.90-0.83 (m, 2H), 0.63-0.59 (m, 2H).

### [Example 4]

### Synthesis of 4'-(cyclopropanecarbonyl)-N-cyclopropyl-6-methyl-[1,1'-biphenyl]-3-carboxamide (compound 4)

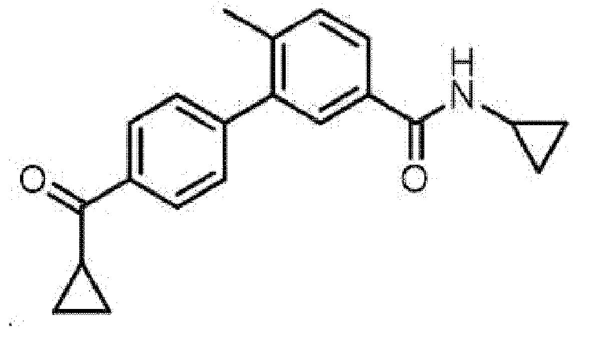

Intermediate compound (4-bromophenyl)(cyclopropyl)methanone (compound 17b) (42 mg, 0.188 mmol) was used as the starting material to finally obtain compound 4 through the same method as for compound 1 (27 mg, 40%); ¹H-NMR (400 MHz, CDCl₃) δ 8.07 (d, J = 8.2 Hz, 2H), 7.67 (dd, J = 8.0, 1.8 Hz, 1H), 7.61 (d, J = 1.8 Hz, 1H), 7.41 ¹H-NMR (400 MHz, CDCl₃) δ 8.07 (d, J = 8.2 Hz, 2H), 7.67 (dd, J = 8.0, 1.8 Hz, 1H), 7.61 (d, J = 1.8 Hz, 1H), 7.41(d, J = 8.2 Hz, 2H), 7.33 (d, J = 8.0 Hz, 1H), 6.35 (s, 1H), 2.93-2.87 (m, 1H), 2.75-2.69 (m, 1H), 2.29 (s, 3H), 1.29-1.24 (m, 2H), 1.10-1.05 (m, 2H), 0.88-0.83 (m, 2H), 0.63-0.59 (m, 2H); LR-MS (ESI⁺) m/z (M+H)⁺ 320.2; HR-MS (ESI⁺) calculated for (M+H)⁺ 320.1651; found 320.1613.

### [Example 5]

### Synthesis of 4'-(cyclobutanecarbonyl)-N-cyclopropyl-6-methyl-[1,1'-biphenyl]-3-carboxamide (compound 5)

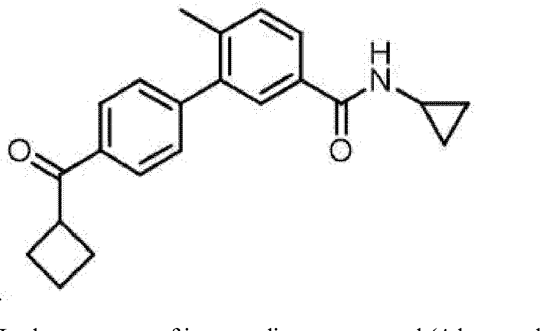

In the presence of intermediate compound (4-bromophenyl)(cyclobutyl)methanone (compound 17c)(67 mg, 0.280 mmol), N-cyclopropyl-4-methyl-3-(4,4,5,5-tetramethyl-1,3,2-dioxaborolan-2-yl)benzamide (110 mg, 0.420 mmol), tetrakis(triphenylphosphine)palladium (33 mg, 0.028 mmol), and potassium carbonate (77 mg, 0.560 mmol) were dissolved in dimethylformamide (3 mL) and heated to 120°C. When it is observed that the starting material disappeared by thin-layer chromatography, the temperature was lowered to room temperature, the reaction was stopped using 2N-hydrochloric acid, filtration was performed using celite, and then the filtrate was extracted with ethyl acetate and water. Magnesium sulfate was added to the obtained organic layer to perform dehydration, and then the solvent of the filtrate obtained by filtering under reduced pressure was removed and concentrated. Compound 5 was then finally obtained by column chromatography (56 mg, 59%); ¹H-NMR (400 MHz, CDCl₃) δ 7.93 (d, J = 8.2 Hz, 2H), 7.66 (dd, J = 7.9, 1.8 Hz, 1H), 7.58 (d, J = 1.8 Hz, 1H), 7.37 (d, J = 8.2 Hz, 2H), 7.31 (d, J = 7.9 Hz, 1H), 6.36 (s, 1H), 4.07-3.98 (m, 1H), 2.92-2.86 (m, 1H), 2.50-2.40 (m, 2H), 2.37-2.29 (m, 2H), 2.28 (s, 3H), 2.16-2.07 (m, 1H), 1.97-1.89 (m, 1H), 0.87-0.82 (m, 2H), 0.62-0.58 (m, 2H); LR-MS (ESI⁺) m/z (M+H)⁺ 334.2; HR-MS (ESI⁺) calculated for (M+H)⁺ 334.1807; found 320.1771.

### [Example 6]

### Synthesis of 4'-(cyclopentanecarbonyl)-N-cyclopropyl-6-methyl-[1,1'-biphenyl]-3-carboxamide (compound 6)

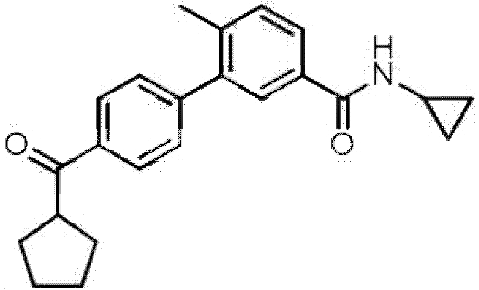

Intermediate compound (4-bromophenyl)(cyclopentyl)methanone (compound 17d)(31 mg, 0.102 mmol) was used as the starting material to finally obtain compound 6 through the same method as for compound 1 (11 mg, 30%); ¹H-NMR (400 MHz, CDCl₃) δ 8.03 (d, J = 8.3 Hz, 2H), 7.66 (dd, J = 7.9, 1.8 Hz, 1H), 7.59 (d, J = 1.7 Hz, 1H), 7.40 (d, J = 8.2 Hz, 2H), 7.33 (d, J = 7.9 Hz, 1H), 6.26 (s, 1H), 3.75 (quin, J = 7.9 Hz, 1H), 2.93-2.87 (m, 1H), 2.29 (s, 3H), 1.99-1.94 (m, 4H), 1.79-1.65 (m, 4H), 0.89-0.84 (m, 2H), 0.62-0.60 (m, 2H); LR-MS (ESI⁺) m/z (M+H)⁺ 348.2; HR-MS (ESI⁺) calculated for (M+H)⁺ 348.1964; found 348.1931.

### [Example 7]

### Synthesis of 4'-(cyclohexanecarbonyl)-N-cyclopropyl-6-methyl-[1,1'-biphenyl]-3-carboxamide (compound 7)

.

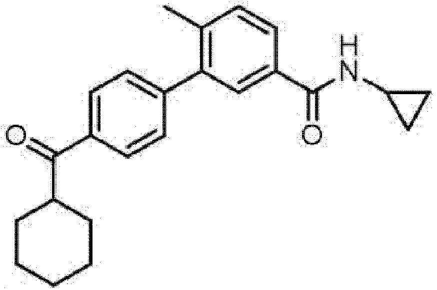

Intermediate compound (4-bromophenyl)(cyclohexyl)methanone (compound 17e)(66 mg, 0.250 mmol) was used as the starting material to finally obtain compound 7 through the same method as for compound 5 (44 mg, 50%); ¹H-NMR (400 MHz, DMSO-d₆) δ 8.00 (d, J = 8.4 Hz, 2H), 7.66 (dd, J = 8.0, 1.9 Hz, 1H), 7.58 (d, J = 1.8 Hz, 1H), 7.40 (d, J = 8.4 Hz, 2H), 7.33 (d, J = 8.0 Hz, 1H), 6.23 (s, 1H), 3.34-3.26 (m, 1H), 2.93-2.87 (m, 1H), 2.29 (s, 3H), 1.95-1.86 (m, 4H), 1.59-1.46 (m, 4H), 1.44-1.40 (m, 2H), 0.89-0.84 (m, 2H), 0.63-0.59 (m, 2H); LR-MS (ESI⁺) m/z (M+H)⁺ 362.2; HR-MS (ESI⁺) calculated for (M+H)⁺ 362.2120; found 362.2102.

### [Example 8]

### Synthesis of 4'-(cycloheptanecarbonyl)-N-cyclopropyl-6-methyl-[1,1'-biphenyl]-3-carboxamide (compound 8)

.

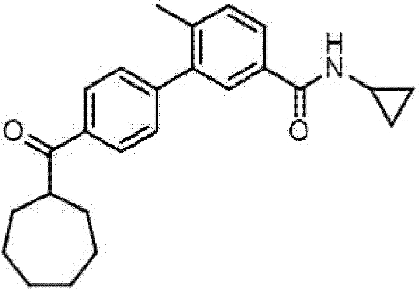

Intermediate compound (4-bromophenyl) cycloheptylmethanone (compound 17f)(20.0 mg, 0.071 mmol) was used as the starting material to finally obtain compound 8 through the same method as for compound 5 (18.5 mg, 58%); ¹H-NMR (400 MHz, CDCl₃) δ 7.98 (d, J = 8.4 Hz, 2H), 7.69-7.57 (m, 2H), 7.39 (d, J = 8 Hz, 2H), 7.32 (d, J = 8 Hz, 1H), 6.38 (bs, 1H), 3.50-3.41 (m, 1H), 2.92-2.84 (m, 1H), 2.28 (s, 3H), 2.00-1.51 (m, 12H), 0.88-0.80 (m, 2H), 0.64-0.56 (m, 2H).

### [Example 9]

### Synthesis of 4'-(cyclopent-3-enecarbonyl)-N-cyclopropyl-6-methyl-[1,1'-biphenyl]-3-carboxamide (compound 9)

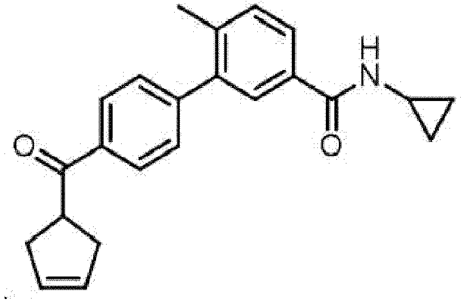

Intermediate compound (4-bromophenyl)(cyclopent-3-en-1-yl)methanone (compound 20)(54 mg, 0.215 mmol) was used as the starting material to finally obtain compound 9 through the same method as for compound 5 (37 mg, 50%); ¹H-NMR (400 MHz, CDCl₃) δ 8.02 (d, J = 8.2 Hz, 2H), 7.67 (dd, J = 7.9, 1.9 Hz, 1H), 7.60 (d, J = 1.8 Hz, 1H), 7.41 (d, J = 8.3Hz, 2H), 7.33 (d, J = 8.0 Hz, 1H), 6.37 (s, 1H), 5.71 (s, 2H), 4.15-4.07 (m, 1H), 2.93-2.87 (m, 1H), 2.85-2.70 (m, 4H), 2.29 (s, 3H), 0.88-0.83 (m, 2H), 0.63-0.59 (m, 2H); LR-MS (ESI⁺) m/z (M+H)⁺ 346.2; HR-MS (ESI⁺) calculated for (M+H)⁺ 346.1807; found 346.1771.

### [Example 10]

### Synthesis of N-cyclopropyl-4'-(3-hydroxycyclopentanecarbonyl)-6-methyl-[1,1'-biphenyl]-3-carboxamide (compound 10)

.

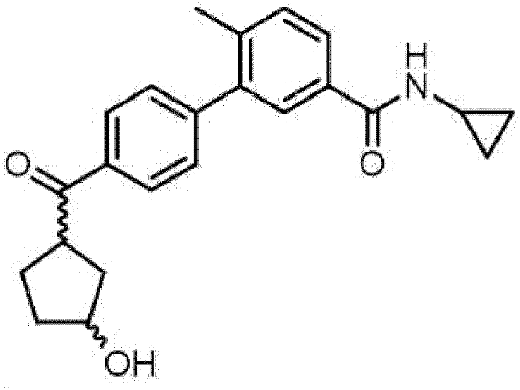

Intermediate compound (4-bromophenyl)(3-hydroxycyclopentyl)methanone (compound 21)(25 mg, 0.093 mmol) was used as the starting material to obtain compound 10 in a diastereoisomer mixture state through the same method as for compound 5 (31 mg, 72%); ¹H-NMR (400 MHz, CDCl₃) δ 8.05 (d, J = 8.3 Hz, 2H), 7.69-7.65 (m, 1H), 4.53-4.51 (m, 0.7H) + 4.40-3.37 (m, 0.3H), 4.02-3.94 (m, 0.7H) + 3.90-3.84 (m, 0.3H), 2.24-2.08 (m, 2H), 1.98-1.82 (m, 3H), 1.77-1.72 (m, 1H); LR-MS (ESI⁺) m/z (M+H)⁺ 364.2; HR-MS (ESI⁺) calculated for (M+H)⁺ 364.1913; found 364.1898.

### [Example 11]

### Synthesis of N-cyclopropyl-4'-(3-(2-hydroxyethoxy)cyclopentanecarbonyl)-6-methyl-[1,1'-biphenyl]-3-carboxamide (compound 11)

.

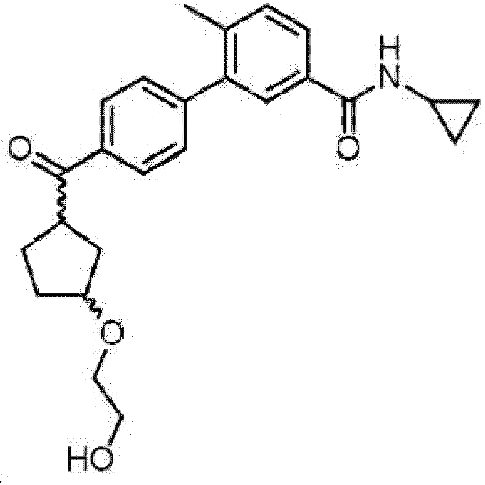

Intermediate compound (4-bromophenyl)(3-(2-hydroxyethoxy)cyclopentyl)methanone (compound 22)(181 mg, 0.579 mmol) was used as the starting material to obtain compound 11 in a diastereoisomer mixture state through the same method as for compound 1 (133 mg, 56%); LR-MS (ESI⁺) m/z (M+H)⁺ 408.2; HR-MS (ESI⁺) calculated for (M+H)⁺ 408.2175; found 408.2132.

### [Example 12]

### Synthesis of N-cyclopropyl-4'-(3-(2-methoxyethoxy)cyclopentane-1-carbonyl)-6-methyl-[1,1'-biphenyl]-3-carboxamide (compound 12)

.

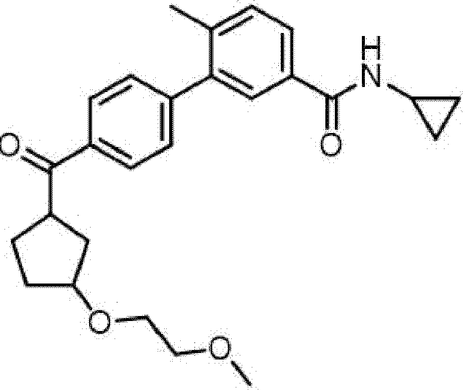

Using (4-bromophenyl)(3-(2-methoxyethoxy)cyclopentyl)methanone (96.7 mg, 0.296 mmol) as the starting material, N-cyclopropyl-4-methyl-3-(4,4,5,5-tetramethyl-1,3,2-dioxabololan-2-yl)benzamide (134 mg, 0.444 mmol), tetrakis(triphenylphosphine)palladium (35 mg, 0.030 mmol), and potassium carbonate (123 mg, 0.888 mmol) were dissolved in isopropyl alcohol (3.5 mL) and heated to 90°C. When it was observed that the starting material disappeared by thin-layer chromatography, the temperature was lowered to room temperature, the reaction was stopped using 2N-hydrochloric acid, filtration was performed using celite, and then the filtrate was extracted with ethyl acetate and water. Magnesium sulfate was added to the obtained organic layer to perform dehydration, and then the solvent of the filtrate obtained by filtering under reduced pressure was removed and concentrated. Compound 12 was then finally obtained by column chromatography (12 mg, 10%); 1H-NMR (400 MHz, CDCl3) δ 8.03 (d, J = 8.2 Hz, 2H), 7.68-7.65 (m, 1H), 7.58 (d, J = 1.68 Hz, 1H), 7.42-7.40 (m, 2H), 7.33 (d, J = 7.9 Hz, 1H), 6.25 (s, 1H), 4.11-4.10 (m, 1H), 3.99-3.98 (m, 1H), 3.61-3.55 (m, 4H), 3.41 (s, 3H), 2.91-2.90 (m, 1H), 2.29-2.27 (s, 3H), 2.19-2.04 (m, 4H), 1.91-1.85 (m, 2H), 0.89-0.83 (m, 2H), 0.62-0.61 (m, 2H).

### [Example 13]

### Synthesis of N-cyclopropyl-4'-(2-hydroxypropanoyl)-6-methyl-[1,1'-biphenyl]-3-carboxamide (compound 13)

.

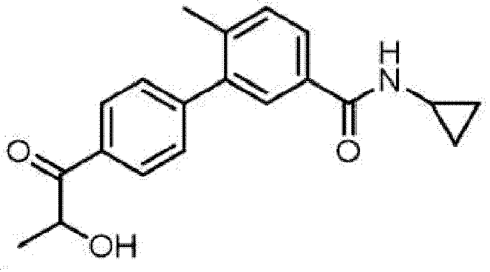

Intermediate compound N-cyclopropyl-6-methyl-4'-propionyl-[1,1'-biphenyl]-3-carboxamide (26.0 mg, 0.085 mmol) and copper(II) bromide (2 mg, 0.1eq) were dissolved in 1 ml of DMSO, and a reaction was carried out at 90°C after argon gas substitution. When it was observed that the starting material disappeared by thin-layer chromatography, the reaction was terminated by diluting with dichloromethane and water. After performing extraction with dichloromethane and water, the organic layer was dehydrated with magnesium sulfate, and the solvent of the filtrate obtained by filtering under reduced pressure was concentrated and removed. Compound 13 was then finally obtained by column chromatography (7.7 mg, 28%); ¹H-NMR (400 MHz, CDCl₃) δ 7.99 (d, J = 8 Hz, 2H), 7.66 (dd, J = 7.8 Hz, 1H), 7.61 (m, 1H), 7.45 (d, J = 8.4 Hz, 2H), 7.34 (d, J = 8 Hz, 1H), 6.25 (ds, 1H), 3.82-3.78 (m, 1H), 2.93-2.86 (m, 1H), 2.30 (s, 3H), 1.50 (d, J = 7.2 Hz, 3H), 0.90-0.84 (m, 2H), 0.63-0.59 (m, 2H).

### [Example 14]

### Synthesis of N-cyclopropyl-6-methyl-4'-(pyrrolidine-3-carbonyl)-[1,1'-biphenyl]-3-carboxamide (compound 14)

.

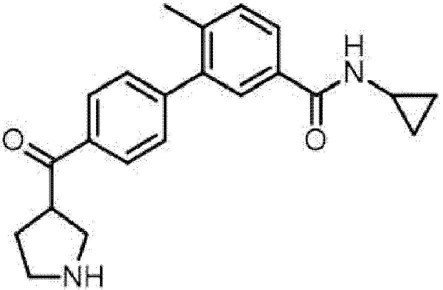

Intermediate compound tert-butyl 3-(5'-(cyclopropylcarbamoyl)-2'-methyl-[1,1'-biphenyl]-4-carbonyl)pyrrolidine-1-carboxylate (103 mg, 0.230 mmol) was dissolved in dichloromethane (5 mL), trifluoroacetic acid (1 mL) was added dropwise to perform a reaction at room temperature. When it was observed that the starting material disappeared by thin-layer chromatography, the solvent was completely removed by raising the temperature, and hydrochloric acid (2.0 M in ether) was added to form a precipitate. After washing using ether, the precipitate was dissolved in an aqueous solution saturated with sodium bicarbonate to make it basic, and then extraction was performed with ethyl acetate. Magnesium sulfate was added to the obtained organic layer to perform dehydration, and then the solvent of the filtrate obtained by filtering under reduced pressure was removed and concentrated. Compound 14 was then finally obtained by column chromatography (33mg, 41%); ¹H-NMR (400 MHz, CDCl₃ + MeOD) δ 8.11 (d, J = 8.3 Hz, 2H), 7.75-7.72 (m, 1H), 7.70 (d, J = 1.6 Hz, 1H), 7.52 (d, J = 8.3 Hz, 2H), 7.37 (d, J = 8.0 Hz, 1H), 4.34-4.29 (m, 1H), 3.65-3.61 (m, 1H), 3.47-3.42 (m, 1H), 3.35-3.33 (m, 1H), 3.32-3.25 (m, 2H), 2.87-2.84 (m, 1H), 2.50-2.43 (m, 1H), 2.32 (s, 3H), 2.22-2.16 (m, 1H), 0.84-0.79 (m, 2H), 0.68-0.64 (m, 2H).

### [Example 15]

### Synthesis of N-cyclopropyl-4'-(1-(2-methoxyethyl)pyrrolidine-3-carbonyl)-6-methyl-[1,1'-biphenyl]-3-carboxamide (compound 15)

.

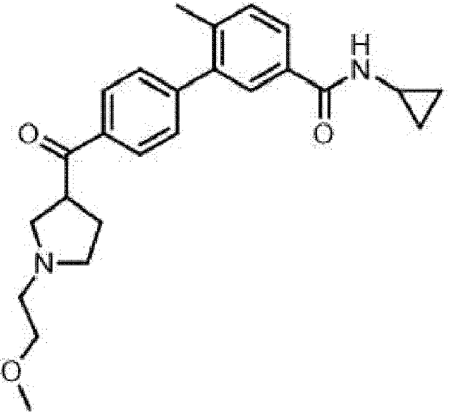

Intermediate compound N-cyclopropyl-6-methyl-4'-(pyrrolidine-3-carbonyl)-[1,1'-biphenyl]-3-carboxamide (compound 14) (19 mg, 0.055 mmol) and potassium carbonate (15 mg, 0.110 mmol) were dissolved in dimethylformamide (2 mL), and 2-bromoethyl methyl ether (0.008 mL) was added dropwise. It was heated to a reaction temperature of 50° C. If it was observed that the starting material disappeared by thin-layer chromatography, the temperature was lowered to room temperature, and dilution was performed using ethyl acetate. Magnesium sulfate was added to the obtained organic layer by performing extraction with ethyl acetate and water so as to perform dehydration, and then the solvent of the filtrate obtained by filtering under reduced pressure was removed and concentrated. Compound 15 was then finally obtained by column chromatography (4 mg, 19%); ¹H-NMR (400 MHz, CDCl₃) δ 8.01 (d, J = 8.2 Hz, 2H), 7.68-7.65 (m, 1H), 7.59 (d, J = 1.8 Hz, 1H), 7.42 (d, J = 8.2 Hz, 2H), 7.34 (d, J = 7.9 Hz, 1H), 6.22 (s, 1H), 4.11-4.04 (m, 1H), 3.57 (t, J = 5.4 Hz, 2H), 3.38 (s, 3H), 3.28 (bs, 1H), 3.07 (bs, 1H), 2.93-2.87 (m, 1H), 2.80 (bs, 2H), 2.57-2.51 (m, 1H), 2.29 (s, 3H), 2.25-2.20 (m, 1H), 2.04-2.00 (m, 2H), 0.89-0.83 (m, 2H), 0.63-0.59 (m, 2H).

### [Experiment Example 1]

### Evaluation of lipid affinity via Calculated LogP (CLogP)

Using Chemdaw software, the structural formulas of compounds 1 to 15 were input, and the CLogP values were identified, whose results are shown in Table 1 below.

**TABLE 1**

| Compound Number | CLogP |
|---|---|
| 1 | 2.8 |
| 2 | 3.7 |
| 3 | 3.7 |
| 4 | 3.6 |
| 5 | 4.0 |
| 6 | 4.5 |
| 7 | 5.1 |
| 8 | 5.7 |
| 9 | 4.0 |
| 10 | 3.3 |
| 11 | 3.0 |
| 12 | 3.6 |
| 13 | 2.5 |
| 14 | 2.8 |
| 15 | 3.5 |

According to Lipinski's rule of five, which was written by examining existing drugs, if the CLogP is less than 5, it has characteristics of drugs that are easy to be orally administered. Thus, it can be seen that compounds 1 to 15 may be developed as drugs for oral administration.

### [Experiment Example 2]

### Evaluation of inhibitory ability of p38α mitogen-activated protein kinase (MAPK)

To identify the ability to inhibit p38 MAPK, compounds 1 to 15 were diluted to identify the inhibitory effect according to concentration. Briefly, the compounds were diluted at concentrations of 1000, 333, 111, 37.0, 12.3, 4.12, 1.37, 0.457, 0.152, and 0.0495 nM, respectively, and then a peptide/kinase mixture (final 7.07-28.3 ng p38 alpha, 2 µM Serine/Threonine 15, 50 mM HEPES pH 7.0, 0.01% NaN3) and ATP solution were added. After carrying out a reaction at room temperature for 1 hour, a development reagent was added, and a reaction was performed again at room temperature for 1 hour. Kinase inhibitory ability was identified at 445 nm and 520 nm wavelengths, whose results are shown in Table 2 below.

**TABLE 2**

| Compound Number | IC₅₀(nM) |
|---|---|
| 1 | 71.8 |
| 2 | 21.9 |
| 3 | 50.7 |
| 4 | 39.2 |
| 5 | 86.7 |
| 6 | 128 |
| 7 | 72.6 |
| 8 | 46.6 |
| 9 | 46.5 |
| 10 | 22.5 |
| 11 (syn isomer) | 27.8 |
| 11 (anti isomer) | 58.4 |
| 12 | 21.8 |
| 13 | 50.8 |
| 14 | 126 |
| 15 | 158 |

It was found that most of the compounds of the present disclosure were potent inhibitors that inhibit p38 alpha by 50% at concentrations less than equal to 100 nM.

### [Experimental Example 3]

### Evaluation of p38 MAPK protein inhibitory ability in cells

To identify inhibition ofp-p38 MAPK, inhibition of p38 MAPK was identified after each compound was treated to Raw264.7 cells obtained by transforming macrophages into cancerous cells that cause inflammation according to p-p38 MAPK activity. Briefly, Raw264.7 cells were cultured in Dulbecco's modified Eagle Medium supplemented with 10% fetal bovine serum and penicillin/streptomycin (100 U/ml). Raw 264.7 cells were treated with 10 µM of compounds 1 to 15 according to the present disclosure in a petri dish respectively, followed by culture in a CO₂ incubator for 24 hours. The effect of each compound on inhibition of p-p38 MAPK in cells was identified through protein immunoblot.

After completion of cell culture under the experimental conditions, washing was performed with cold phosphate buffered saline (PBS). The cells were then collected and centrifuged at 1200 rpm for 3 minutes. The precipitate was dissolved in cells with 2X sample buffer (1M Tris-HCl pH 6.8, 50% glycerol, 10% SDS, 2-mercaptoethanol, 1% bromophenol blue) and boiled at 100°C for 10 minutes. The prepared protein samples were subjected to sodium dodecyl sulfate poly acrylamide gel electrophoresis and then transferred to PVDF membrane (Millipore). Blocking was performed in a 5% bovine serum albumin (BSA, RMbio) solution for a specific coupling reaction between the protein on the PVDF membrane and antibody. The BSA solution was then discarded, and the membrane was washed with PBS-T (0.5% tween 20 in PBS). The coupling reaction between the protein on the membrane and antibody was carried out overnight at 4°C, followed by washing again with PBS-T. Finally, a secondary antibody (Cell signaling Technology) conjugated with horseradish peroxidase (HRP) was diluted in PBS-T, and a reaction was carried out at room temperature for 2 hours. Luminata Forte HRP substrate (Millipore) was used to determine the protein amount on the membrane.

The results are shown in Table 3 below.

**TABLE 3**

| Compound Number | p-p38 inhibition % |
|---|---|
| 4 | 20.9 |
| 5 | 80.4 |
| 6 | 76.2 |
| 7 | 87.6 |
| 8 | 83.0 |
| 9 | 81.7 |
| 10 | 44.7 |
| 11 (syn isomer) | 41.9 |
| 11 (anti isomer) | 64.1 |
| 14 | 88.6 |
| 15 | 79.2 |

As a result of the immunoblot, it was found that the synthesized compound of the present disclosure effectively inhibits p38 phosphorylation in cells at a concentration of 10 µM.

### [Experiment Example 4]

### Evaluation of nitric oxide inhibitory ability according to cellular inflammatory response

In order to identify an inflammatory inhibitory effect upon inhibition of p-p38 MAPK, each compound was treated to Raw264.7 cells obtained by transforming macrophages into cancerous cells involved in the inflammatory response, so as to identify inhibition of inflammation due to inhibition of p38 MAPK. Briefly, Raw264.7 cells were cultured in Dulbecco's Modified Eagle Medium supplemented with 10% fetal bovine serum and penicillin/streptomycin (100 U/ml). Raw 264.7 cells were treated with 10 µM of compounds 1 to 15 according to the present disclosure in a petri dish respectively, followed by culture in a CO₂ incubator for 24 hours.

After completion of cell culture under experimental conditions, the supernatant was obtained and centrifuged at 1200 rpm for 3 minutes. The centrifuged supernatant was mixed with Griess reagent (1% sulfanilamide, 0.1% N-(1-naphthyl)ethylenediamine dihydrochloride, 5% phosphoric acid) at 1:1 and then a reaction was performed for 10 minutes under shaded condition. The concentration of nitric oxide was determined at a wavelength of 550 nm.

The results are shown in Table 4 below.

**TABLE 4**

| Compound Number | NO inhibition (%) |
|---|---|
| 4 | 26.0 |
| 5 | 11.9 |
| 6 | 24.0 |
| 7 | 15.5 |
| 8 | 43.6 |
| 9 | 21.1 |
| 10 | 11.1 |
| 11 (syn isomer) | 30.6 |
| 11 (anti isomer) | 11.0 |

It has been found that the compounds of the present disclosure inhibit generation of nitric oxide, which is a final product of the inflammatory response, and the compounds of the present disclosure inhibit the inflammatory response through p38 inhibition.

## Claims

1. A compound represented by the following Chemical Formula 1 or a pharmaceutically acceptable salt thereof; 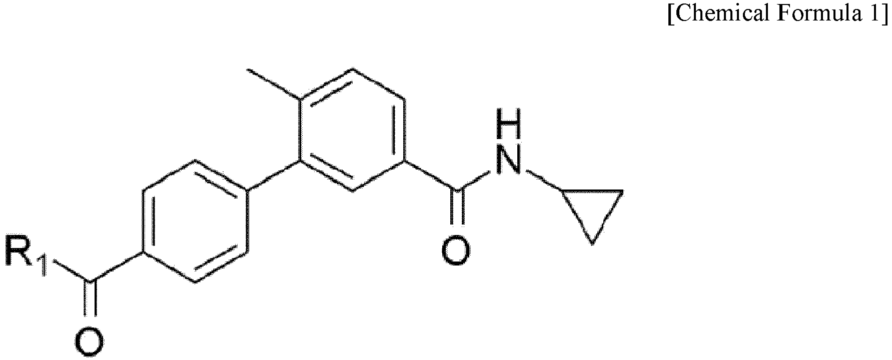 wherein, in the above Chemical Formula,
R₁ is -C₁-C₆alkyl-OH; -C₁-C₆alkyl-O-C₁-C₆alkyl; C₃-C₇cycloalkyl substituted or unsubstituted with -OH, -O-(CH₂)m-OH, or -O-(CH₂)m-O-C₁-C₆alkyl; C₃-C₇cycloalkenyl; or pyrrolidine substituted or unsubstituted with -C₁-C₆alkylC₁-C₆alkoxy.

2. A compound selected from the group consisting of the following compounds or a pharmaceutically acceptable salt thereof:
| No. | Chemical name | Structural formula |
|---|---|---|
| 1 | N-cyclopropyl-4'-(2-hydroxy-2-methylpropanoyl)-6-methyl-[1,1'biphenyl]-3-carboxamide | 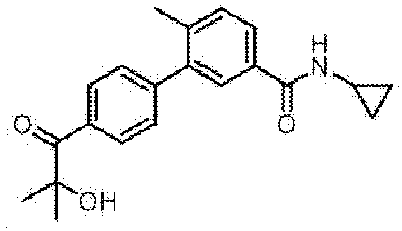 |
| 2 | N-cyclopropyl-4'-(4-methoxy-2-methylbutanoyl)-6-methyl-[1,1'-biphenyl]-3-carboxamide | 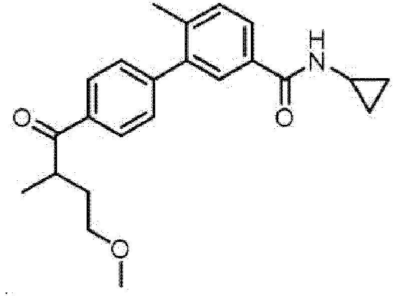 |
| 3 | N-cyclopropyl-4'-(5-methoxy-2-methylpentanoyl)-6-methyl-[1,1'-biphenyl]-3-carboxamide | 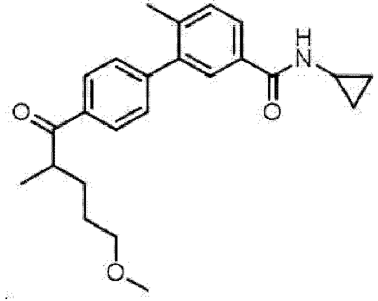 |
| 4 | 4'-(cyclopropanecarbonyl)-N-cyclopropyl-6-methyl-[1,1'-biphenyl]- 3-carboxamide | 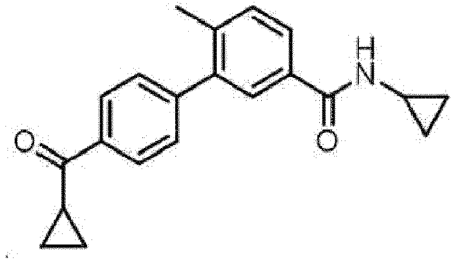 |
| 5 | 4'-(cyclobutanecarbonyl)-N-cyclopropyl-6-methyl-[1,1'-biphenyl]- 3-carboxamide | 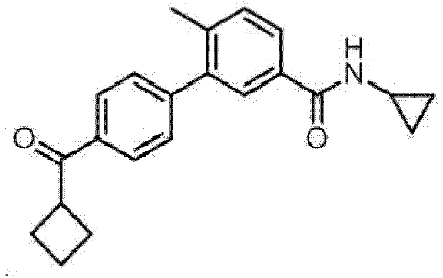 |
| 6 | 4'-(cyclopentanecarbonyl)-N-cyclopropyl-6-methyl-[1,1'-biphenyl]- 3-carboxamide | 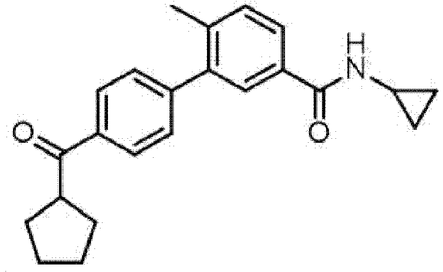 |
| 7 | 4'-(cyclohexanecarbonyl)-N-cyclopropyl-6-methyl-[1,1'-biphenyl]- 3-carboxamide | 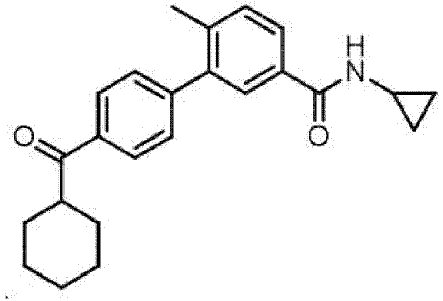 |
| 8 | 4'-(cycloheptanecarbonyl)-N-cyclopropyl-6-methyl-[1,1'-biphenyl]- 3-carboxamide | 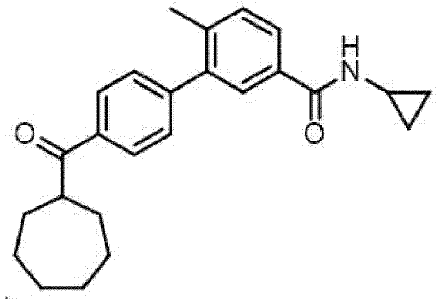 |
| 9 | 4'-(cyclopent-3-enecarbonyl)-N-cyclopropyl-6-methyl-[1,1'-biphenyl]- 3-carboxamide | 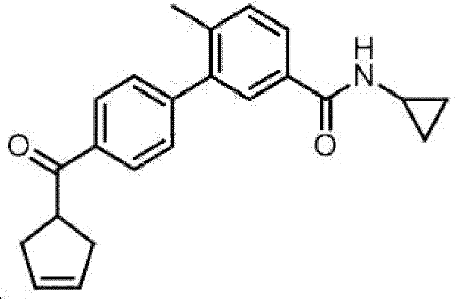 |
| 10 | N-cyclopropyl-4'-(3-hydroxycyclopentanecarbonyl)-6-methyl-[1,1'-biphenyl]-3-carboxamide | 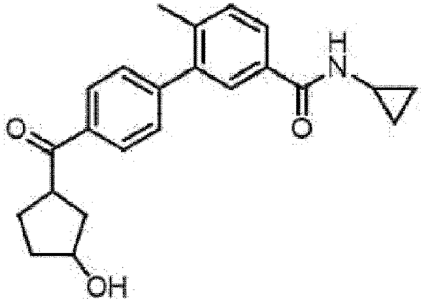 |
| 11 | N-cyclopropyl-4'-(3-(2-hydroxyethoxy)cyclopentanecarbonyl) -6-methyl-[1,1'-biphenyl]-3- carboxamide | 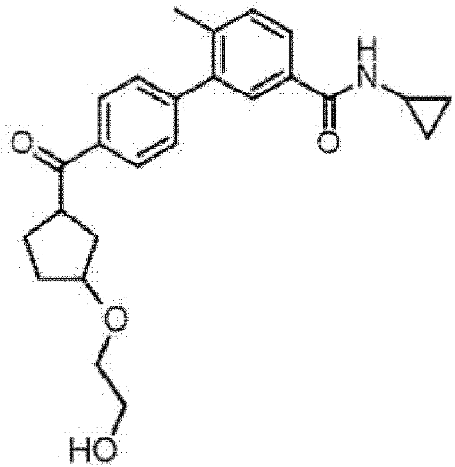 |
| 12 | N-cyclopropyl-4'-(3-(2-methoxyethoxy)cyclopentane-1- carbonyl)-6-methyl-[1,1'-biphenyl]-3-carboxamide | 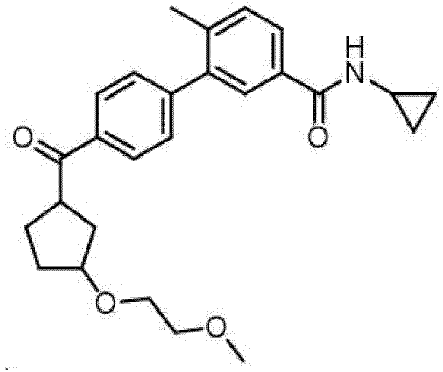 |
| 13 | N-cyclopropyl-4'-(2-hydroxypropanoyl)-6-methyl-[1,1'-biphenyl]-3-carboxamide | 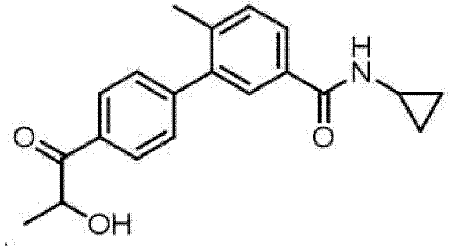 |
| 14 | N-cyclopropyl-6-methyl-4'-(pyrrolidine-3-carbonyl)-[1,1'-biphenyl]-3-carboxamide | 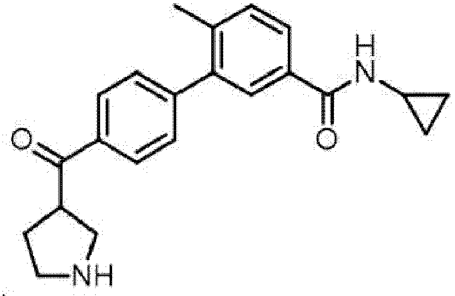 |
| 15 | N-cyclopropyl-4'-(1-(2-methoxyethyl)pyrrolidine-3-carbonyl)-6-methyl-[1,1'-biphenyl]-3-carboxamide | 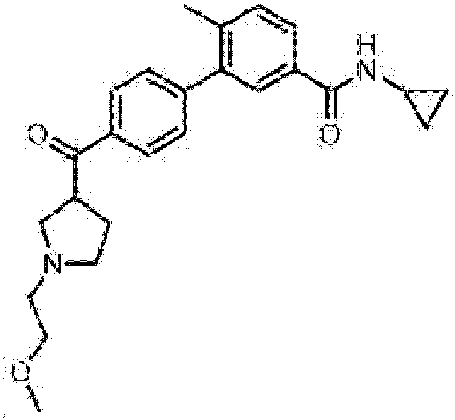 |
